# EUROPEAN PATENT APPLICATION

(11) **EP 4 218 923 A1**
(43) Date of publication of application: **02.08.2023**
(21) Application number: 21785746.5
(22) Date of filing: 16.09.2021
(51) Int. Cl.: A61N 2/02, A61N 2/00

(54) **MAGNETIC-FIELD-GENERATING THERAPY DEVICE**

(30) Priority: 24.09.2020 KR 20200123932
(71) Applicant: Remed Co., Ltd., Daejeon 34025 (KR)
(72) Inventor: YOON, Se Jin, Anyang-si, Gyeonggi-do 14055 (KR); KIM, Ghi Young, Anyang-si, Gyeonggi-do 14055 (KR)
(74) Representative: ABG Intellectual Property Law, S.L.
(86) International application number: PCT/KR2021/012666
(87) International publication number: WO 2022/065800

(57) **Abstract**

The present invention relates to a treatment device using a magnetic field, which includes a module-type functional cushion that is couplable to a sitting type treatment device generating a pulse magnetic field.

The treatment device using the magnetic field in accordance with an embodiment of the present invention includes: a magnetic field generating coil for generating a magnetic field that is applied to a body of a user; a blower for supplying a fluid for cooling the magnetic field generating coil; a duct for guiding the fluid between the blower and the magnetic field generating coil; and a functional cushion disposed on an area in which the magnetic field is generated by the magnetic field generating coil in a couplable manner and having a shape on which buttocks of the user is seated.

## Description

### TECHNICAL FIELD

The present invention disclosed herein relates to a treatment device using a magnetic field, and more particularly, to a treatment device using a magnetic field, which includes a module-type functional cushion that is couplable to a sitting type treatment device generating a pulse magnetic field.

### BACKGROUND ART

Urinary incontinence is caused such that muscles that control the pelvic floor muscles are deflected downward instead of being fixed due to muscle weakness caused by hormonal changes of menopause and nerve damages due to pregnancy, childbirth, or a surgery. Also, prostate hypertrophy is a symptom in which a prostate of a urinary system gradually increases in size. When the prostate hypertrophy is worsened, the prostate presses a urethra to damage a bladder and a kidney, thereby generating a fatal complication such as uremia.

A physical treatment method for the urinary incontinence and the prostate hypertrophy may be classified into a non-surgical treatment such as a pelvic exercise and a surgical treatment such as electromagnetic stimulation. Typically, the non-surgical treatment method is classified into an insertion type and a sitting type according to a shape of a treatment instrument for the urinary incontinence.

In general, the sitting type treatment device operates in a method of inducing a magnetic field by applying a pulse-type current to a coil and stimulating an affected area by inducing a current caused by the induced magnetic field into a human tissue. The treatment device using a magnetic field includes a coil generating a magnetic field by a current applied from a main body and generates a magnetic field having a desired intensity by applying a current of several thousands amperes for a short time (50µs to 300µs).

However, since the sitting type device that is the related art operates in a method of applying a magnetic field to a wide area of buttocks of a user, stimulation is generally applied over the entire buttocks, and thus the stimulation to a perineum has a weak intensity. Therefore, an effect of the stimulation to the perineum is insignificant.

Since the above-described limitations of the related art still exist in a non-surgical treatment device for the urinary incontinence, a measure for solving the limitations is required.

### DISCLOSURE OF THE INVENTION

### TECHNICAL PROBLEM

The present invention provides a treatment device using a magnetic field, which is capable of increasing a treatment effect by allowing a generated magnetic field to be exactly applied toward a perineum of a user.

The present invention also provides a treatment device using a magnetic field, which includes a module-type functional cushion to select a functional cushion suitable for each individual according to a body condition of a user.

The present invention also provides a treatment device using a magnetic field, which increases convenience by including a detachable functional cushion.

The present invention also provides a treatment device using a magnetic field, which is more expandable than a sitting type usage method by allowing a magnetic field to be applied to various portions of a body.

### TECHNICAL SOLUTION

In accordance with an embodiment of the present invention, a treatment device using a magnetic field includes: a magnetic field generating coil configured to generate a magnetic field that is applied to a body of a user; a blower configured to supply a fluid for cooling the magnetic field generating coil; a duct configured to guide the fluid between the blower and the magnetic field generating coil; and a functional cushion disposed on an area in which the magnetic field is generated by the magnetic field generating coil in a couplable manner and having a shape on which buttocks of the user is seated.

Also, the functional cushion may include an opening that allows the magnetic field generated from the magnetic field generating coil to be applied directly to the body of the user.

Also, the functional cushion may include a left pelvis support and a right pelvis support, which respectively support a left pelvis and a right pelvis of the user, and a left thigh support and a right thigh support, which respectively support a left thigh and a right thigh of the user.

Also, the left pelvis support and the right pelvis support may be connected to each other, the left thigh support may extend from the left pelvis support, the right thigh support may extend from the right pelvis support, and the opening may be defined between the left thigh support and the right thigh support.

Also, the left thigh support and the right thigh support may be spaced apart from each other so that the opening is opened in a direction in which each of the left thigh support and the right thigh support extends.

Also, the opening may extend toward the left pelvis support and the right pelvis support, and the left pelvis support and the right pelvis support may be at least partially spaced apart from each other in an area in which the opening is defined.

Also, ends of the left thigh support and the right thigh support, which are disposed at a side opposite to the left pelvis support and the right pelvis support, may be connected to each other.

Also, the left pelvis support and the right pelvis support may be spaced apart from each other, the left thigh support may extend from the left pelvis support, the right thigh support may extend from the right pelvis support, the openings may be respectively defined between the left pelvis support and the right pelvis support and between left thigh support and the right thigh support, and the treatment device may further include a connection part configured to connect the left thigh support and the right thigh support between the openings.

Also, the opening may be defined in an area corresponding to a perineum of the user. Also, the functional cushion may be coupled onto a frame of the treatment device using the magnetic field by using a clamp or a Velcro.

### ADVANTAGEOUS EFFECTS

The treatment device using the magnetic field of the present invention may induce the user to sit at the exact position through the functional cushion, so that the magnetic field generated from the magnetic field generating coil is exactly applied to the perineum of the user.

Also, the treatment device using the magnetic field of the present invention may allow various users to select the functional cushion suitable for each individual according to various body conditions of the users by including the module-type functional cushion. Also, the treatment device using the magnetic field of the present invention may allow the functional cushion to be coupled or removed according to the convenience of the user by including the detachable functional cushion.

Also, the treatment device using the magnetic field of the present invention may be used as the treatment device that is more expandable than the sitting type usage method by forming the functional cushion suitable for other body portions such as arms and legs of the user in addition to the buttocks.

### BRIEF DESCRIPTION OF THE DRAWINGS

FIG 1 is a cross-sectional view illustrating a treatment device using a magnetic field of the present invention.
FIG 2 is a schematic conceptual view illustrating a position at which an inlet blower is disposed in accordance with the present invention.
FIG 3 is a schematic conceptual view illustrating a position at which an outlet blower is disposed in accordance with the present invention.
FIGS. 4A and 4B are a cross-sectional view for comparing and explaining a cooling structure of present invention.
FIG 5 is an enlarged cross-sectional view illustrating area A of FIG 1.
FIG 6 is a cross-sectional view illustrating a state in which a functional cushion is coupled to the treatment device using the magnetic field.
FIGS. 7A to 7C are a perspective view illustrating a C-type functional cushion that is couplable to the treatment device using the magnetic field of the present invention.
FIG. 8 is a perspective view illustrating an O-type functional cushion in accordance with another embodiment.
FIG 9 is a perspective view illustrating a H-type functional cushion in accordance with another embodiment.

### MODE FOR CARRYING OUT THE INVENTION

Advantages and features of the present invention, and implementation methods thereof will be clarified through following embodiments described with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present disclosure to those skilled in the art. Further, the present disclosure is only defined by scopes of claims.

It will be understood that although the terms of first and second are used herein to describe various elements, these elements should not be limited by these terms. These terms are only used to distinguish one component from another component. Accordingly, a first component that will be described below may be a second component within the technical idea of the present disclosure.

The meaning of "include," "comprise," "including," or "comprising," specifies a property, a region, a fixed number, a step, a process, an element and/or a component but does not exclude other properties, regions, fixed numbers, steps, processes, elements and/or components.

Also, for convenience of description, the dimensions of elements are exaggerated or downscaled. In the drawings, the thicknesses of layers and regions are exaggerated for clarity, and thus the present invention is not limited thereto.

Like reference numerals refer to like elements throughout.

It will also be understood that when an element is referred to as being "'connected to" or "engaged with" another element, it can be directly connected to the other element, or intervening elements may also be present. It will also be understood that when an element is referred to as being 'directly connected to' another element, there is no intervening elements.

It will also be understood that when a layer is referred to as being "on" another layer or substrate, it can be directly on the other layer or substrate, or intervening layers may also be present. In contrast, when an element is referred to as being "directly on" another element or layer, there are no intervening elements or layers present.

Spatially relative terms, such as "below", "beneath", "lower", "above", "upper" and the like, may be used herein for ease of description to describe one element or feature's relationship to another element(s) or feature(s) as illustrated in the figures. The spatially relative terms should be understood as terms which include different directions of configurative elements in addition to directions illustrated in the figures when using or operating the present invention.

Features of various embodiments of the present disclosure are partially or entirely coupled or combined with each other, and technically various interlocking and driving are enabled. Also, the embodiments may be independently performed with respect to each other, or performed in combination of each other.

Hereinafter, a treatment device using a magnetic field of the present invention will be described in detail with reference to the accompanying drawings.

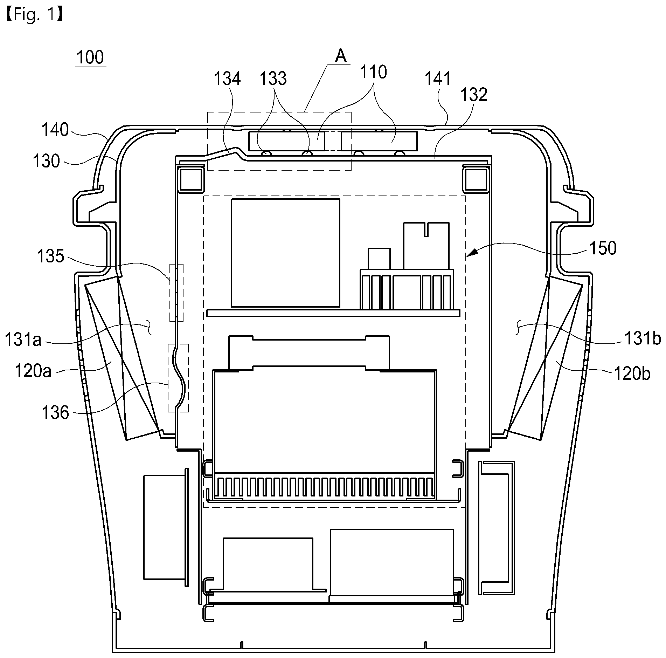

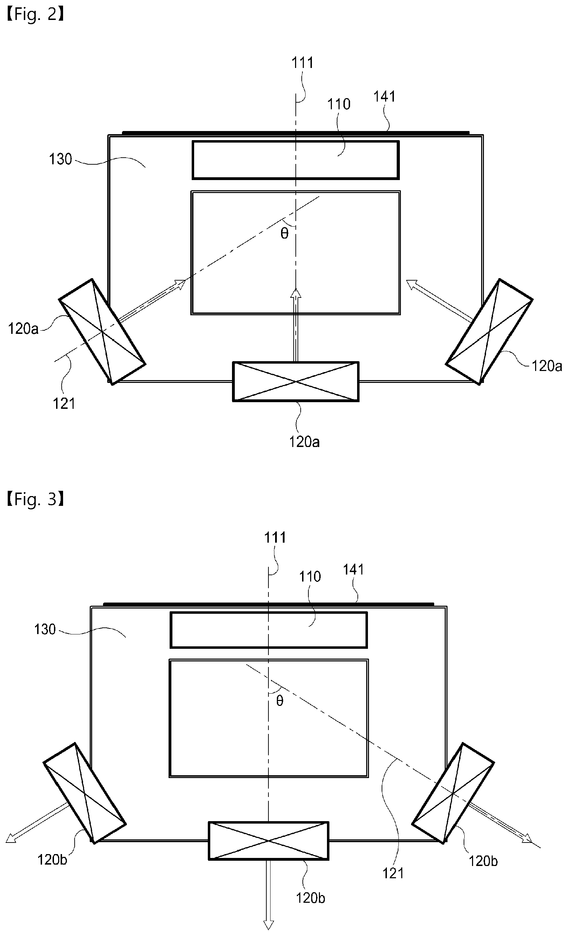

FIG 1 is a cross-sectional view illustrating a treatment device using a magnetic field of the present invention, FIG 2 is a schematic conceptual view illustrating a position of an inlet blower in accordance with the present invention, FIG 3 is a schematic conceptual view illustrating a position of an outlet blower in accordance with the present invention,

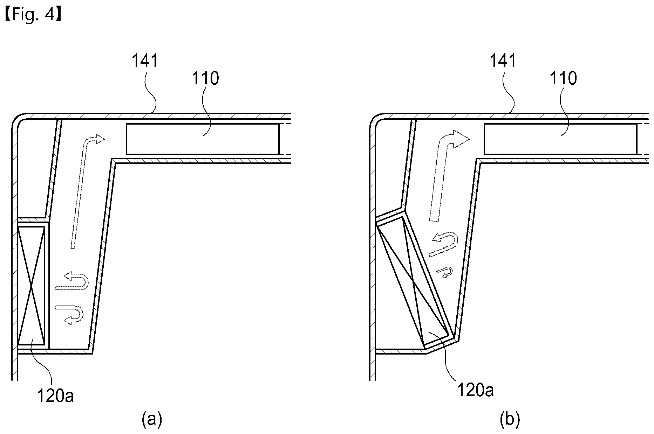

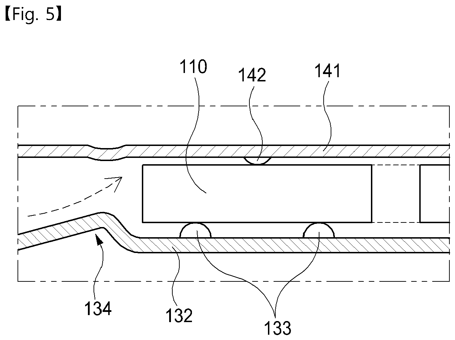

FIG 4 is a cross-sectional view for explaining by comparing a cooling structure of the present invention, and FIG 5 is an enlarged cross-sectional view illustrating area A of FIG 1.

Referring to FIG 1, a treatment device 100 using a magnetic field (hereinafter, referred to as a magnetic field generating treatment device 100) of the present invention includes a magnetic field generating coil 110, a blower 120, a duct 130 for guiding a fluid, a housing 140, and an internal electronic device 150. Here, since the above-described components are not essential for implementing the magnetic field generating treatment device 100, the magnetic field generating treatment device 100 may include more or less components than the above-described components. Also, each of the components may be constituted as a separate chip, module, or device, or contained in one device.

The magnetic field generating coil 110 generates a pulse magnetic field through an applied current. The magnetic field generating coil 110 is disposed below a close contact surface 141 that closely contacts a portion of a body.

The blower 120 supplies a fluid for cooling the magnetic field generating coil 110. Referring to FIG 1, the blower 120 includes an inlet blower 120a supplying a fluid to the magnetic field generating coil 110 and an outlet blower 120b exhausting the fluid passed through the magnetic field generating coil 110 to the outside.

The inlet blower 120a and the outlet blower 120b may be respectively disposed at positions that are symmetric to each other with respect to the magnetic field generating coil 110.

The inlet blower 120a supplies a fluid from the outside to the inside of the magnetic field generating treatment device 100 of the present invention. Here, the inlet blower 120a may be one selected from a fan, a blower, a compressor, and a pump. Referring to FIG 2, the inlet blower 120a is disposed below the magnetic field generating coil 110. The inlet blower 120a is disposed so that an angle θ between a fluid supply direction 121 from the inlet blower 120a and a central axis 111 of the magnetic field generating coil 110 is equal to or less than a right angle.

The outlet blower 120b exhausts a fluid flowing in the magnetic field generating treatment device 100. Here, the outlet blower 120b may be one selected from a fan, a blower, a compressor, and a pump. Referring to FIG 3, the outlet blower 120b is disposed below the magnetic field generating coil 110. The outlet blower 120b is disposed so that an angle θ between a fluid supply direction 121 from the outlet blower 120b and the central axis 111 of the magnetic field generating coil 110 is equal to or less than a right angle.

Here, the angle θ between the fluid supply direction 121 and the central axis 111 of the magnetic field generating coil 110 may be greater than 0° and less than 90°, preferably equal to or greater than 10° and equal to or less than 60°.

Advantages of forming a predetermined angle between the fluid supply direction 121 from the inlet blower 120a and the central axis 111 of the magnetic field generating coil 110 will be described with reference to FIGS. 4A and 4B.

As the angle θ between the fluid supply direction 121 from each of the inlet blower 120a and the outlet blower 120b and the central axis 111 of the magnetic field generating coil 110 is set to a preset angle, the magnetic field generating treatment device 100 may improve a cooling efficiency of the magnetic field generating coil 110 instead of arranging the blower 120 on the same plane as the magnetic field generating coil 110.

FIG 4A is a view illustrating a case when the angle between the fluid supply direction 121 from the inlet blower 120a and the central axis 111 of the magnetic field generating coil 110 is 90°. In this case, the fluid supplied from the inlet blower 120a is not supplied toward the magnetic field generating coil 110 but returned to the inlet blower 120a by an inner circumferential surface of the duct 130. Since this arrangement does not effectively move the fluid supplied from the inlet blower 120a to the magnetic field generating coil 110, the cooling efficiency of the magnetic field generating treatment device 100 may be degraded. However, FIG 4B is a view illustrating a case when the fluid supply direction 121 from the inlet blower 120a forms a predetermined angle with the central axis 111 of the magnetic field generating coil 110. In this case, since the fluid supplied from the inlet blower 120a heads toward the magnetic field generating coil 110, the magnetic field generating treatment device 100 has an advantage in that the fluid supplied from the inlet blower 120a may be effectively moved to the magnetic field generating coil 110.

Furthermore, as the angle θ between the fluid supply direction 121 from each of the inlet blower 120a and the outlet blower 120b and the central axis 111 of the magnetic field generating coil 110 is set to the preset angle, an overall volume of the magnetic field generating treatment device 100 of the present invention may be reduced. As illustrated in FIG 1, as a groove is defined in an area of the housing 140 between the blower 120 and the magnetic field generating coil 110, a user may use the groove as a handle to thus improve usage convenience. Also, as the angle θ between the fluid supply direction 121 from each of the inlet blower 120a and the outlet blower 120b and the central axis 111 of the magnetic field generating coil 110 is set to the preset angle, a limitation of introducing dusts at a bottom portion into the device may be prevented although the inlet blower 120a adopts one of a fan, a blower, a compressor, and a pump. Referring to FIG 1, the duct 130 guides the fluid between the blower 120 and the magnetic field generating coil 110.

Specifically, the duct 130 has one end 131a opened to the inlet blower 120a and the other end 131b opened to the outlet blower 120b. The duct 130 guides the fluid introduced from the inlet blower 120a to pass through the magnetic field generating coil 110 and be discharged through the outlet blower 120b.

The duct 130 is formed such that an internal cross-section of an area in which the magnetic field generating coil 110 is disposed is less than a cross-section of each of the one end 131a and the other end 131b. In other words, the duct 130 is formed such that a cross-section of an area in which a seated surface 132 is provided is less than that of each of the one end 131a and the other end 131b. Since a velocity of the fluid increases in an area in which the internal cross-section decreases, the duct 130 has a structure in which the velocity of the fluid increases in the area in which the magnetic field generating coil 110 is disposed.

The duct 130 includes the seated surface 132 on which the magnetic field generating coil 110 is seated at a position corresponding to the close contact surface 141.

The magnetic field generating coil 110 is seated on the seated surface 132, and the duct 130 includes support 133 supporting the magnetic field generating coil 110 at a position corresponding to the seated position. The support 133 allows the magnetic field generating coil 110 to be spaced a predetermined distance from the inner circumferential surface of the duct 130. The fluid supplied from the inlet blower 120a flows through the space spaced by the support 133.

The support 133 may be rubber. The support 133 may serve to absorb a vibration generated in an operation process of the magnetic field generating coil 110.

Referring to FIGS. 1 and 5, the duct 130 includes a protruding portion 134 protruding from the seated surface 132 toward the magnetic field generating coil 110. The protruding portion 134 is provided in at least a portion of the seated surface 132 provided between the inlet blower 120a and the magnetic field generating coil 110.

As illustrated in FIG 5, the protruding portion 134 guides the fluid introduced from the inlet blower 120a to flow toward an upper portion of the magnetic field generating coil 110. In other wards, the protruding portion 134 guides the fluid introduced from the inlet blower 120a to flow toward an area between the magnetic field generating coil 110 and the close contact surface 141. The protruding portion 134 may improve the cooling efficiency of the portion closely contacting a portion of the body of the user by increasing a flow velocity and a flow rate supplied to the area between the magnetic field generating coil 110 and the close contact surface 141.

Although the protruding portion 134 is formed by bending a portion of the inner circumferential surface of the duct 130 in the present invention, the embodiment of the present invention is not limited thereto. For example, the protruding portion 134 may be formed in various methods such as a method of attaching a separate member to a portion of the inner circumferential surface of the duct 130.

Referring to FIG 1, the duct 130 includes a hole 135 defined in at least a portion of an area between the inlet blower 120a and the magnetic field generating coil 110.

The hole 135 allows a portion of the fluid introduced to the duct 130 to pass therethrough. The fluid passed through the hole 135 may cool the internal electronic device 150. As the duct 130 includes the hole 135, a portion of the fluid introduced from the inlet blower 120a may be used to cool heat generated from the magnetic field generating coil 110, and the rest thereof may be used to cool heat generated from the internal electronic device 150. Here, the number and the size of the hole 135 may be determined so that about 70% of the fluid supplied to the hole 135 flows to the magnetic field generating coil 110, and about 30% thereof flows to the internal electronic device 150.

The duct 130 may simultaneously cool the magnetic field generating coil 110 and the internal electronic device 150 by including the hole 135. Through this, the magnetic field generating treatment device 100 of the present invention may increase the cooling efficiency through a compact structure thereof instead of including a separate cooling unit for the internal electronic device 150.

Referring to FIG 1, the duct 130 may include a curved portion 136 obtained by bending at least a portion of the inner circumferential surface between the inlet blower 120a and the magnetic field generating coil 110.

Although the curved portion 136 is formed by bending a portion of the inner circumferential surface of the duct 130 in the present invention, the embodiment of the present invention is not limited thereto. For example, the curved portion 136 may have an embossing shape or be formed in various methods such as a method of attaching a separate member to a portion of the inner circumferential surface of the duct 130.

The duct 130 may reduce a noise generated by using a fan or a blower by including the curved portion 136. A noise generated when the fluid introduced from the inlet blower 120a contacts the curved portion 136 of the duct 130 may be less than that generated when the fluid introduced from the inlet blower 120a contacts other areas of the duct 130.

Referring to FIG 1, the close contact surface 141 includes a support 142 protruding from a bottom surface of the close contact surface 141 to the magnetic field generating coil 110. Here, the close contact surface 141 may be a portion of the housing 140 or a portion of the duct 130.

The support 142 allows the magnetic field generating coil 110 to be spaced a predetermined distance from the close contact surface 141. The fluid supplied from the inlet blower 120a flows through the space spaced by the support 142. The support 142 may be rubber like the support 133 provided on the seated surface 132. The support 142 may serve to absorb a vibration generated in an operation process of the magnetic field generating coil 110.

The magnetic field generating treatment device 100 of the present invention may include functional cushion 160, 170, and 180.

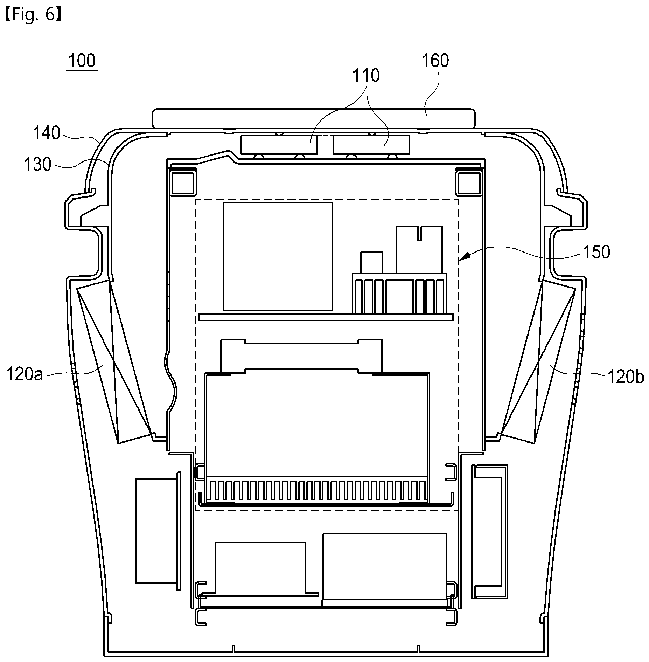

FIG 6 is a cross-sectional view illustrating a state in which a functional cushion is coupled to the magnetic field generating treatment device 100 of the present invention,

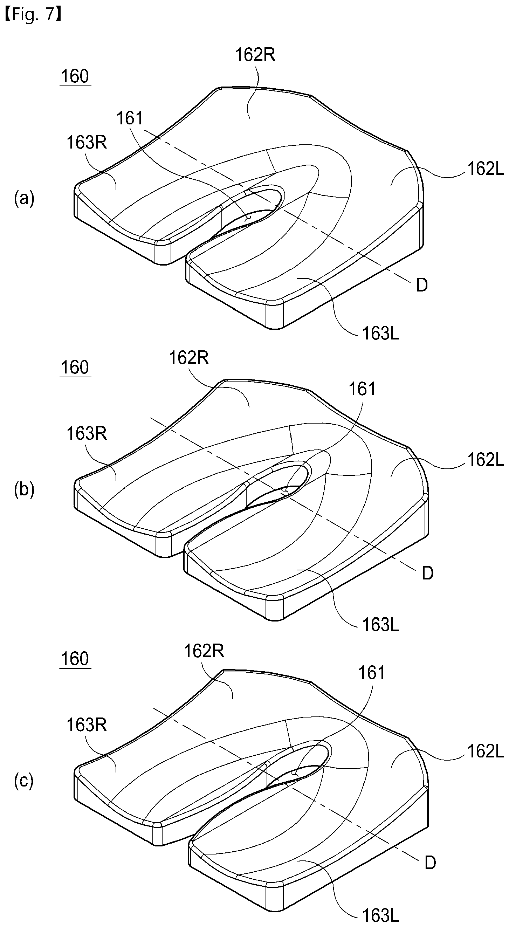

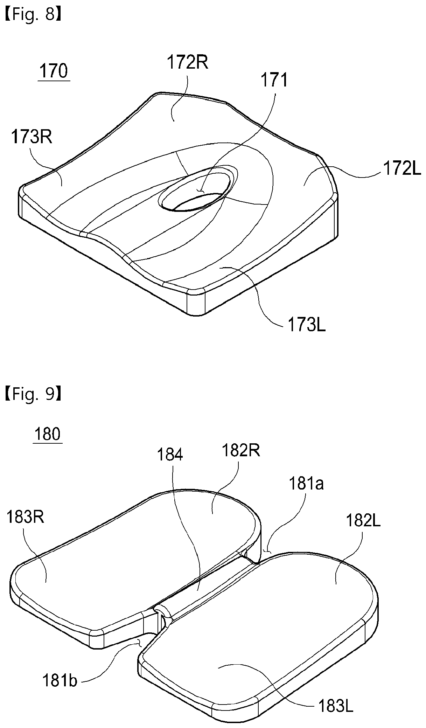

FIG 7 is a perspective view illustrating a C-type functional cushion that is couplable to the magnetic field generating treatment device 100 of the present invention, FIG. 8 is a perspective view illustrating an O-type functional cushion in accordance with another embodiment, and FIG 9 is a perspective view illustrating a H-type functional cushion in accordance with another embodiment.

Referring to FIG 6, the functional cushion 160 induces the user to sit at an exact position of the magnetic field generating treatment device 100 of the present invention so that a magnetic field generated from the magnetic field generating coil 110 is exactly applied to a perineum of the user.

The functional cushion 160 may be disposed on an area in which the magnetic field is generated by the magnetic field generating coil 110, e.g., the close contact surface 141, in a couplable manner. Here, the functional cushion 160 may be coupled onto a frame of the magnetic field generating treatment device 100 by using a clamp or a Velcro.

The functional cushion 160 may have a shape on which buttocks of the user is seated.

The shape on which the buttocks of the user are seated may include various embodiments.

Firstly, referring to FIGS 7A to 7C illustrating the C-type functional cushion, the functional cushion 160 include an opening 161. The opening 161 is defined in an area corresponding to the perineum of the user. The opening 161 is opened toward the close contact surface 141 and the perineum of the user so that the magnetic field generated from the magnetic field generating coil 110 is applied directly to the body of the user.

The functional cushion 160 includes a left pelvis support 162L and a right pelvis support 162R, which respectively support a left pelvis and a right pelvis of the user. Also, the functional cushion 160 includes a left pelvis support 162L and a right pelvis support 162R, which respectively support a left pelvis and a right pelvis of the user. The left pelvis support 162L and the right pelvis support 162R of the C-type functional cushion 160 may be connected to each other. Also, a left thigh support 163L extends from the left pelvis support 162L, and a right thigh support 163R extends from the right pelvis support 162R.

The left pelvis support 162L and the right pelvis support 162R supporting a pelvis of the user and the left thigh support 163L and the right thigh support 163R supporting a thigh of the user may not refer to physically divided areas although areas thereof may be conceptually divided by a dotted line 'D' illustrated in FIG 7.

In the functional cushion 160 illustrated in FIG 7A, the opening 161 is defined between the left thigh support 163L and the right thigh support 163R. Here, the left thigh support 163L and the right thigh support 163R are spaced apart from each other so that the opening 161 is opened in a direction in which each of the left thigh support 163L and the right thigh support 163R extends.

In the functional cushion 160 illustrated in FIG 7A, the opening 161 is defined from an end of each of the left thigh support 163L and the right thigh support 163R to an area defined by the dotted line D.

Alternatively, in the functional cushion 160 illustrated in FIG 7B or 7C, the opening 161 extends further toward the left pelvis support 162L and the right pelvis support 162R in comparison with the functional cushion 160 illustrated in FIG 7A, and the left pelvis support 162L and the right pelvis support 162R are at least partially spaced apart from each other in the area in which the opening 161 is defined.

In the functional cushion 160 illustrated in FIG 7B or 7C, the opening 161 may extend from the end of each of the left thigh support 163L and the right thigh support 163R to a portion of an area of each of the left pelvis support 162L and the right pelvis support 162R through the area defined by the dotted line D.

All of the functional cushions 160 illustrated in FIGS. 7A to 7C have similar overall shapes as the C-type cushion, but are different in depth of the opening 161. A position and a posture of the user sitting on the functional cushion 160 are changed according to a shape of the opening 161. Thus, one of the functional cushions 160 illustrated in FIGS. 7A to 7C may be selectively used according to a body condition of the user or a position, to which the magnetic field is preferably applied, of the user.

For example, a user who wants to increase a treatment effect on a position of a prostate may select the functional cushion 160 illustrated in FIG 7C. In the functional cushion 160 illustrated in FIG 7C, the opening 161 is relatively biased to a rear portion. When the user sits on the functional cushion 160 of FIG 7C, the user may have a posture of sitting backward from a central portion of the magnetic field generating treatment device 100 of the present invention In this case, the prostate disposed at a front of the perineum may be close to a central portion of the magnetic field generating coil 110. Thus, the user may use the magnetic field generating treatment device 100 of the present invention so that the magnetic field is applied to be more concentrate on the prostate, thereby increasing a treatment effect on the prostate.

However, this is merely illustrative, and the embodiment of the present invention is not limited thereto. For example, the user may select the functional cushion suitable for each individual according to a body condition such as a shape of buttocks and a sitting posture of the user.

Referring to FIG. 8 illustrating the O-type functional cushion, the functional cushion 170 includes an opening 171. The opening 171 is defined in an area corresponding to the perineum of the user. The opening 171 is opened toward the close contact surface 141 and the perineum of the user so that the magnetic field generated from the magnetic field generating coil 110 is applied directly to the body of the user.

The functional cushion 170 includes a left pelvis support 172L and a right pelvis support 172R, which respectively support the left pelvis and the right pelvis of the user. Also, the functional cushion 170 includes a left thigh support 173L and a right thigh support 173R, which respectively support the left thigh and the right thigh of the user. The left pelvis support 172L and the right pelvis support 172R of the O-type functional cushion 170 may be connected to each other. Also, the left thigh support 173L extends from the left pelvis support 172L, and the right thigh support 173R extends from the right pelvis support 172R.

The opening 171 is defined between the left thigh support 173L and the right thigh support 173R. Here, as ends of the left thigh support 173L and the right thigh support 173R, which are disposed at an opposite side of the left pelvis support 172L and the right pelvis support 172R, are connected to each other, an O-shape is formed unlike the C-type functional cushion 160 of FIG 7.

The O-type functional cushion 170 is a modified example of the C-type functional cushion 160. Thus, although not shown in the drawing, the opening 171 of the O-type functional cushion 170 may be changed in such a manner that the opening 161 of the C-type functional cushion 160 is deformed as in FIGS. 7A to 7C. The user may select one of the functional cushions 170 having the openings 171 having various shapes according to a body condition or a position to which the magnetic field is applied. Referring to FIG 9 illustrating the H-type functional cushion, the functional cushion 180 includes openings 181a and 181b. Each of the openings 181a and 191b is defined in an area corresponding to the perineum of the user. Each of the openings 181a and 181b is opened toward the close contact surface 141 and the perineum of the user so that the magnetic field generated from the magnetic field generating coil 110 is applied directly to the body of the user.

The functional cushion 180 includes a left pelvis support 182L and a right pelvis support 182R, which respectively support the left pelvis and the right pelvis of the user. Also, the functional cushion 180 includes a left thigh support 183L and a right thigh support 183R, which respectively support the left thigh and the right thigh of the user. The left pelvis support 182L and the right pelvis support 182R of the H-type functional cushion 180 are spaced apart from each other unlike the above-described C-type functional cushion 160 and the O-type functional cushion 170. Also, the left thigh support 183L extends from the left pelvis support 182L, and the right thigh support 183R extends from the right pelvis support 182R.

The openings 181a and 181b are respectively defined between the left pelvis support 182L and the right pelvis support 182R and between the left thigh support 183L and the right thigh support 183R. Here, the functional cushion 180 has an H-shape by including a connection part 184 for connecting the left thigh support 183L and the right thigh support 183R between the openings 181a and 181b.

The magnetic field generating treatment device 100 of the present invention may include a functional cushion suitable for other body portions such as arms and legs of the user in addition to the buttocks. In this case, the magnetic field generating treatment device 100 of the present invention may be used as a treatment device that is expendable from a sitting type usage method.

The magnetic field generating treatment device of the present invention may induce the user to sit at an exact position through the functional cushion, so that the magnetic field generated from the magnetic field generating coil is exactly applied to the perineum of the user. Also, the magnetic field generating treatment device of the present invention may allow various users to select the functional cushion suitable for each individual according to various body conditions of the users by including the module-type functional cushion. Also, the magnetic field generating treatment device of the present invention may allow the functional cushion to be coupled or removed according to convenience of the user by including the detachable functional cushion.

The description of the present invention is intended to be illustrative, and those with ordinary skill in the technical field of the present invention will be understood that the present invention can be carried out in other specific forms without changing the technical idea or essential features. Thus, the above-disclosed embodiments are to be considered illustrative and not restrictive.

## Claims

1. A treatment device using a magnetic field, comprising:
a magnetic field generating coil configured to generate a magnetic field that is applied to a body of a user;
a blower configured to supply a fluid for cooling the magnetic field generating coil;
a duct configured to guide the fluid between the blower and the magnetic field generating coil; and
a functional cushion disposed on an area in which the magnetic field is generated by the magnetic field generating coil in a couplable manner and having a shape on which buttocks of the user is seated.

2. The treatment device of claim 1, wherein the functional cushion comprises an opening that allows the magnetic field generated from the magnetic field generating coil to be applied directly to the body of the user.

3. The treatment device of claim 2, wherein the functional cushion comprises a left pelvis support and a right pelvis support, which respectively support a left pelvis and a right pelvis of the user, and a left thigh support and a right thigh support, which respectively support a left thigh and a right thigh of the user.

4. The treatment device of claim 3, wherein the left pelvis support and the right pelvis support are connected to each other,
the left thigh support extends from the left pelvis support, and the right thigh support extends from the right pelvis support, and
the opening is defined between the left thigh support and the right thigh support.

5. The treatment device of claim 4, wherein the left thigh support and the right thigh support are spaced apart from each other so that the opening is opened in a direction in which each of the left thigh support and the right thigh support extends.

6. The treatment device of claim 5, wherein the opening extends toward the left pelvis support and the right pelvis support, and
the left pelvis support and the right pelvis support are at least partially spaced apart from each other in an area in which the opening is defined.

7. The treatment device of claim 4, wherein ends of the left thigh support and the right thigh support, which are disposed at a side opposite to the left pelvis support and the right pelvis support, are connected to each other.

8. The treatment device of claim 3, wherein the left pelvis support and the right pelvis support are spaced apart from each other,
the left thigh support extends from the left pelvis support, and the right thigh support extends from the right pelvis support,
the openings are respectively defined between the left pelvis support and the right pelvis support and between left thigh support and the right thigh support, and
the treatment device further comprises a connection part configured to connect the left thigh support and the right thigh support between the openings.

9. The treatment device of claim 2, wherein the opening is defined in an area corresponding to a perineum of the user.

10. The treatment device of claim 1, wherein the functional cushion is coupled onto a frame of the treatment device using the magnetic field by using a clamp or a Velcro.
